(19) Europäisches Patentamt
European Patent Office
Office européen des brevets

(11) **EP 2 966 141 A1**

(12) **EUROPEAN PATENT APPLICATION**
published in accordance with Art. 153(4) EPC

(43) Date of publication:
**13.01.2016 Bulletin 2016/02**

(51) Int Cl.:
*C09K 3/18* (2006.01)          *C08G 77/385* (2006.01)
*C08K 3/18* (2006.01)          *C08K 5/098* (2006.01)
*C08K 5/5415* (2006.01)        *C08L 71/00* (2006.01)

(21) Application number: **14759655.5**

(22) Date of filing: **06.03.2014**

(86) International application number:
**PCT/JP2014/055821**

(87) International publication number:
**WO 2014/136896 (12.09.2014 Gazette 2014/37)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **06.03.2013 JP 2013044087
06.03.2013 JP 2013044088
06.03.2013 JP 2013044089**

(71) Applicants:
• **Unimatec Co., Ltd.
Tokyo 105-0012 (JP)**
• **Hirosaki University
Hirosaki-shi, Aomori 036-8560 (JP)**

(72) Inventors:
• **SATO Katsuyuki
Kitaibaraki-shi
Ibaraki 319-1544 (JP)**
• **SAWADA Hideo
Hirosaki-shi
Aomori 036-8560 (JP)**

(74) Representative: **Beckmann, Claus
Kraus & Weisert
Patentanwälte PartGmbB
Thomas-Wimmer-Ring 15
80539 München (DE)**

(54) **FLUORINE-CONTAINING CALCIUM COMPOSITE PARTICLES, PREPARATION METHOD THEREFOR, AND SURFACE TREATMENT AGENT USING SAME AS ACTIVE COMPONENT**

(57) Fluorine-containing calcium composite particles comprising an aggregate of a fluorine-containing alcohol represented by the general formula:

$$C_nF_{2n+1}(CH_2CF_2)_a(CF_2CF_2)_b(CH_2CH_2)_cOH$$

wherein n is an integer of 1 to 6, a is an integer of 1 to 4, b is an integer of 0 to 3, and c is an integer of 1 to 3; and a calcium compound,
or fluorine-containing calcium composite particles comprising a condensate of a fluorine-containing alcohol represented by the general formula: $R_F$-A-OH or the general formula: HO-A-$R_F$'-A-OH wherein $R_F$ is a liner or branched perfluoroalkyl group that may contain an O, S, or N atom, or a polyfluoroalkyl group in which some of the fluorine atoms of the perfluoroalkyl group are replaced by hydrogen atoms; $R_F$' is a linear or branched perfluoroalkylene group containing an O, S, or N atom; and A is an alkylene group having 1 to 6 carbon atoms; alkoxysilane, and a calcium compound. The latter fluorine-containing calcium composite particles are used as an active ingredient of water- and oil-repellent.

**Description**

TECHNICAL FIELD

The present invention relates to fluorine-containing calcium composite particles, a method for producing the particles, and a surface-treating agent comprising the particles as an active ingredient. More particularly, the present invention relates to fluorine-containing calcium composite particles capable of recovering fluorine sources as calcium fluoride and having water- and oil-repellency, a method for producing the particles, and a surface-treating agent comprising the particles as an active ingredient.

BACKGROUND ART

Fluorine sources required for the production of fluorine compounds are produced from fluorite (calcium fluoride). Fluorine compounds produced via various processes using the produced hydrogen fluoride and fluorine gas are applied and developed for various products because they have excellent resistance to chemical and physical changes, such as chemical resistance, heat resistance, and weather resistance, due to their chemical stability. However, the final disposal of fluorine compounds, which are difficult to dispose of, is mostly landfill disposal. This causes problems such as increases in waste and adverse effects on the environment.

Moreover, fluorine-containing organic compounds are known to have water- and oil-repellency derived from their fluorine atoms. For example, Patent Document 1 indicates that a fluorine-containing silane compound of the formula: $C_8F_{17}SO_2R^1(CH_2)_3SiX_3$ is applied to the surface of materials, such as metal, resin, paper, and glass, as a water- and oil-repellent. However, the contact angle of this water- and oil-repellent is suitable for water, but not suitable for liquid paraffin. That is, it is not sufficient as far as oil repellency is concerned.

Patent Document 2 discloses a polymer composition comprising a one-terminal hydrolyzable polymer represented by the general formula:

$$A\text{-Rf-QZa}[(CH_2)_cSi(R_{3-a})Xa]_b$$

Rf: a group represented by $-(CF_2)_d(OC_2F_4)_e(OCF_2)_fO(CF_2)_d-$
A: a monovalent fluorine-containing group having terminal $-CF_2H$
Q: a divalent organic group
Z: a divalent to octavalent organopolysiloxane moiety having a siloxane bond
R: a lower alkyl group or a phenyl group
X: a hydrolyzable group

and a both-terminal hydrolyzable polymer represented by the general formula:

$$Rf\{QZa\,[(CH_2)_cSi(R_{3-a})Xa]_b)_2$$

This polymer composition is described to have good adhesiveness to a substrate and to form a surface coating having excellent water- and oil-repellency, etc.

It is described that a surface-treating agent comprising the polymer composition can be applied to a substrate by any known method, such as brushing, dipping, spraying, or vapor deposition. A vacuum deposition method is used in the Examples of Patent Document 2. However, vacuum deposition requires large-scale processing facilities, and the surface-treating agent is not versatile in this respect. In addition, the oil repellency of the surface-treating agent is equal to or less than that of general fluororesin.

PRIOR ART DOCUMENTS

PATENT DOCUMENTS

Patent Document 1 : JP-A-2-180984

Patent Document 2 : JP-A-2012-233157

Patent Document 3 : JP-B-4674604

Patent Document 4 : WO 2007/080949 A1

Patent Document 5 : JP-A-2008-38015

Patent Document 6 : USP 3,574,770

OUTLINE OF THE INVENTION

PROBLEM TO BE SOLVED BY THE INVENTION

An object of the present invention is to provide fluorine-containing calcium composite particles capable of recovering fluorine sources as calcium fluoride and having water- and oil-repellency, a method for producing the particles, and a surface-treating agent comprising the particles as an active ingredient.

MEANS FOR SOLVING THE PROBLEM

The present invention provides fluorine-containing calcium composite particles comprising an aggregate of a fluorine-containing alcohol represented by the general formula:

$$C_nF_{2n+1}(CH_2CF_2)_a(CF_2CF_2)_b(CH_2CH_2)_cOH \qquad [I]$$

wherein n is an integer of 1 to 6, a is an integer of 1 to 4, b is an integer of 0 to 3, and c is an integer of 1 to 3; and a calcium compound.

The fluorine-containing calcium composite particles are produced by aggregating a fluorine-containing alcohol represented by the above general formula [I] and a calcium compound using an alkaline or acid catalyst.

The present invention also provides fluorine-containing calcium composite particles comprising a condensate of a fluorine-containing alcohol represented by the general formula:

$$R_F\text{-A-OH} \qquad [Ia]$$

or the general formula:

$$HO\text{-A-}R_F'\text{-A-OH} \qquad [Ib]$$

wherein $R_F$ is a liner or branched perfluoroalkyl group that may contain an O, S, or N atom, or a polyfluoroalkyl group in which some of the fluorine atoms of the perfluoroalkyl group are replaced by hydrogen atoms; $R_F'$ is a linear or branched perfluoroalkylene group containing an O, S, or N atom; and A is an alkylene group having 1 to 6 carbon atoms; alkoxysilane, and a calcium compound.

The fluorine-containing calcium composite particles are produced by subjecting a fluorine-containing alcohol represented by the above general formula [Ia] or [Ib] and alkoxysilane to a condensation reaction in the presence of a calcium compound using an alkaline or acid catalyst. The resulting product is used as an active ingredient of surface-treating agents.

EFFECT OF THE INVENTION

The fluorine-containing calcium composite particles of the present invention are novel composite particles. Due to their excellent dispersibility in water and various organic solvents, such as tetrahydrofuran, methanol, isopropanol, 1,2-dichloroethane, dimethylsulfoxide, and dimethylformamide, the fluorine-containing calcium composite particles of the present invention can be applied by a simple surface treatment method, such as dipping. Therefore, when the fluorine-containing calcium composite particles of the present invention are used as a surface-treating agent for glass, metal, stone, resin, and other various substrates, water- and oil-repellency, antifouling function, oil barrier, and other properties derived from their fluorine atoms can be effectively imparted to such substrates. Furthermore, after calcining up to 800°C, calcium fluoride can be detected from the ash content; thus, this calcium fluoride can be recovered and effectively reused as a fluorine source.

EMBODIMENTS FOR CARRYING OUT THE INVENTION

The polyfluoroalkyl alcohol represented by the general formula:

$$C_nF_{2n+1}(CH_2CF_2)_a(CF_2CF_2)_b(CH_2CH_2)_cOH \qquad\qquad [I]$$

n: 1 to 6, preferably 2 to 4
a: 1 to 4, preferably 1
b: 0 to 3, preferably 1 or 2
c: 1 to 3, preferably 1

used in the first invention is disclosed in Patent Document 3, and synthesized through the following series of steps.

First of all, a polyfluoroalkyl iodide represented by the general formula:

$$C_nF_{2n+1}(CH_2CF_2)_a(CF_2CF_2)_b(CH_2CH_2)_cI$$

is reacted with N-methylformamide of the formula: $HCONH(CH_3)$ to form a mixture of polyfluoroalkyl alcohol and its formate. The mixture is then subjected to a hydrolysis reaction in the presence of an acid catalyst to form a fluorine-containing alcohol of the formula:

$$C_nF_{2n+1}(CH_2CF_2)_a(CF_2CF_2)_b(CH_2CH_2)_cOH,$$

which is a polyfluoroalkyl alcohol. Examples of the polyfluoroalkyl iodide include the following:

$$CF_3(CH_2CF_2)(CH_2CH_2)I$$

$$C_2F_5(CH_2CF_2)(CH_2CH_2)I$$

$$C_2F_5(CH_2CF_2)(CH_2CH_2)_2I$$

$$C_3F_7(CH_2CF_2)(CH_2CH_2)I$$

$$C_3F_7(CH_2CF_2)(CH_2CH_2)_2I$$

$$C_4F_9(CH_2CF_2)(CH_2CH_2)I$$

$$C_4F_9(CH_2CF_2)(CH_2CH_2)_2I$$

$$C_2F_5(CH_2CF_2)(CF_2CF_2)(CH_2CH_2)I$$

$$C_2F_5(CH_2CF_2)(CF_2CF_2)(CH_2CH_2)_2I$$

$$C_2F_5(CH_2CF_2)_2(CF_2CF_2)(CH_2CH_2)I$$

$$C_2F_5(CH_2CF_2)_2(CF_2CF_2)(CH_2CH_2)_2I$$

$$C_4F_9(CH_2CF_2)(CF_2CF_2)(CH_2CH_2)I$$

$$C_4F_9(CH_2CF_2)_2(CF_2CF_2)(CH_2CH_2)I$$

$$C_4F_9(CH_2CF_2)(CF_2CF_2)(CH_2CH_2)_2I$$

$$C_4F_9(CH_2CF_2)_2(CF_2CF_2)(CH_2CH_2)_2I$$

An aggregate of such a fluorine-containing alcohol and a calcium compound forms fluorine-containing calcium composite particles.

The aggregation of these components is achieved by stirring them in the presence of a catalyst amount of an alkaline or acid catalyst, such as aqueous ammonia, an aqueous solution of a hydroxide of an alkali metal or alkaline earth metal (e.g., sodium hydroxide, potassium hydroxide, or magnesium hydroxide), hydrochloric acid, or sulfuric acid, at a temperature of about 0 to 100°C, preferably about 10 to 30°C, for about 0.5 to 48 hours, preferably about 1 to 10 hours.

Examples of calcium compounds include inorganic calcium salts, such as calcium hydroxide, calcium oxide, calcium carbonate, calcium chloride, calcium bromide, calcium iodide, calcium nitrate, calcium nitrite, calcium sulfate, calcium sulfite, calcium phosphate, calcium dihydrogen phosphate, calcium pyrophosphate, calcium silicate, calcium borate, and calcium hypochlorite; organic acid calcium salts, such as calcium methanesulfonate, calcium formate, calcium acetate, calcium propionate, calcium 2-ethylhexanoate, calcium stearate, calcium alginate, calcium gluconate, calcium ascorbate, calcium citrate, calcium oxalate, and calcium lactate. When an alkaline calcium compound, such as calcium hydroxide, is used, the compound itself also acts as an alkaline catalyst.

The ratio of these components is such that about 100 to 1,000 parts by weight, preferably about 250 to 500 parts by weight, of fluorine-containing alcohol is used based on 100 parts by weight of the calcium compound. When the ratio of the fluorine-containing alcohol is less than this range, water- and oil-repellency decreases. In contrast, when the ratio of the fluorine-containing alcohol is greater than this range, dispersibility in solvents becomes poor.

The resulting product, i.e., fluorine-containing calcium composite particles, is considered to be obtained by self-aggregation of a micellar aggregate formed by the fluorine-containing alcohol around the calcium compound, which serves as the core. Therefore, the fluorine-containing calcium composite particles effectively exhibit excellent water- and oil-repellency, antifouling properties, and other properties inherent in fluorine. In fact, a glass surface treated with the fluorine-containing calcium composite particles exhibits excellent water- and oil-repellency, and also has the effect of, for example, reducing the weight loss at 800°C. Moreover, the particle size of the fluorine-containing calcium composite particles as well as its variation show small values. The fluorine-containing calcium composite particles are formed as an aggregate of a fluorine-containing alcohol and a calcium compound; however, presence of any other component is allowed as long as the object of the present invention is not impaired.

When a condensation reaction is performed under coexistence of alkoxysilane in the reaction system during the production of such fluorine-containing calcium composite particles, fluorine-containing calcium composite particles can be produced as a condensate comprising three components, i.e., fluorine-containing alcohol, alkoxysilane, and calcium compound.

Examples of the fluorine-containing alcohol represented by the general formula:

$$R_F\text{-A-OH} \qquad \text{[Ia]}$$

$R_F$: a liner or branched perfluoroalkyl group that may contain an O, S, or N atom, or a polyfluoroalkyl group in which some of the fluorine atoms of the perfluoroalkyl group are replaced by hydrogen atoms;
A: an alkylene group having 1 to 6 carbon atoms;

used in the second invention include the following:

$$C_nF_{2n+1}(CH_2CF_2)_a(CF_2CF_2)_b(CH_2CH_2)_cOH \qquad \text{[IIa]}$$

n: 1 to 6, preferably 2 to 4
a: 1 to 4, preferably 1
b: 0 to 3, preferably 1 or 2
c: 1 to 3, preferably 1

$$C_mF_{2m+1}O[CF(CF_3)CF_2O]_dCF(CF_3)(CH_2)_eOH \qquad \text{[IIIa]}$$

m: 1 to 3, preferably 3
d: 0 to 100, preferably 1 to 10
e: 1 to 3, preferably 1

$$C_pF_{2p+1}(CH_2)_qOH \qquad \text{[IVa]}$$

$$C_pF_{2p}H(CH_2)_qOH \qquad \text{[IVa']}$$

p is 1 to 10, preferably 4 to 8
(with the proviso that p is 5 to 10 for surface-treating agent applications)
q is 1 to 6, preferably 2

As the fluorine-containing alcohol represented by the general formula [IIa], the same compound as the fluorine-containing alcohol represented by the general formula [I] is used.

A hexafluoropropene oxide oligomer alcohol represented by the general formula [IIIa]wherein m = 1 and e = 1 is disclosed in Patent Document 4, and synthesized via the following steps.

A fluorine-containing ether carboxylic acid alkyl ester represented by the general formula: $CF_3O[CF(CF_3)CF_2O]_nCF(CF_3)COOR$ (R: an alkyl group, n: an integer of 0 to 12) is reduced by a reducing agent, such as sodium boron hydride.

Examples of the fluorine-containing alcohol represented by the general formula [IVa] include 2,2,2-trifluoroethanol ($CF_3CH_2OH$), 3,3,3-trifluoropropanol ($CF_3CH_2CH_2OH$), 2,2,3,3,3-pentafluoropropanol ($CF_3CF_2CH_2OH$), 3,3,4,4,4-pentafluorobutanol ($CF_3CF_2CH_2CH_2OH$), 2,2,3,3,4,4,5,5,5-nonafluoropentanol ($CF_3CF_2CF_2CF_2CH_2OH$), 3,3,4,4,5,5,6,6,6-nonafluorohexanol ($CF_3CF_2CF_2CF_2CH_2CH_2OH$), 3,3,4,4,5,5,6,6,7,7,8,8,8-tridecafluorooctanol ($CF_3CF_2CF_2CF_2CF_2CF_2CH_2CH_2OH$), and the like.

Examples of the fluorine-containing alcohol represented by the general formula [IVa'] include 2,2,3,3-tetrafluoropropanol ($HCF_2CF_2CH_2OH$), 2,2,3,4,4,4-hexafluorobutanol ($CF_3CHFCF_2CH_2OH$), 2,2,3,3,4,4,5,5-octafluoropentanol ($HCF_2CF_2CF_2CF_2CH_2OH$), and the like.

Moreover, an example of the fluorine-containing alcohol represented by the general formula [Ib] is a perfluoroalkylene ether diol represented by the general formula:

$$HO(CH_2)_fCF(CF_3)[OCF_2CF(CF_3)]_gO(CF_2)_hO[CF(CF_3)CF_2O]_iCF(CF_3)(CH_2)_fOH \qquad [IIb]$$

f: 1 to 3, preferably 1
g + i: 0 to 50, preferably 2 to 50
(with the proviso that g + i is 2 to 50 for surface-treating agent applications)
h: 1 to 6, preferably 2

Fluorine-containing alcohols of the above formula wherein f = 1 are disclosed in Patent Documents 5 and 6, and synthesized via the following series of steps:

$$FOCRfCOF \rightarrow H_3COOCRfCOOCH_3 \rightarrow HOCH_2RfCH_2OH$$

$$Rf: -C(CF_3)[OCF_2C(CF_3)]_aO(CF_2)_cO[CF(CF_3)CF_2O]_bCF(CF_3)-$$

As shown in the above cases, the alkylene group used is an alkylene group having 1 to 6 carbon atoms, preferably a $-CH_2-$ group having 1 carbon atom or a $-CH_2CH_2-$ group having 2 carbon atoms.

A condensate of such a fluorine-containing alcohol, alkoxysilane, and a calcium compound forms fluorine-containing calcium composite particles. The calcium compound to be used may be any of the above-mentioned compounds.

The alkoxysilane to be reacted with a fluorine-containing alcohol forms fluorine-containing calcium composite particles by reaction in the presence of an alkaline or acid catalyst.

The alkoxysilane [III] is, for example, alkoxysilane represented by the general formula:

$$(R_1O)_pSi(OR_2)_q(R_3)_r \qquad [III]$$

$R_1$, $R_3$ : H, $C_1$-$C_6$ alkyl group, or aryl group;
$R_2$: $C_1$-$C_6$ alkyl group or aryl group,
with the proviso that not all of $R_1$, $R_2$, and $R_3$ are aryl groups;
p + q + r: 4, with the proviso that q is not 0

Examples thereof include trimethoxysilane, triethoxysilane, trimethoxymethylsilane, triethoxymethylsilane, trimethoxyphenylsilane, triethoxyphenylsilane, tetramethoxysilane, tetraethoxysilane, and the like.

The reaction between these components is performed in the presence of a catalyst amount of an alkaline or acid catalyst, such as aqueous ammonia, an aqueous solution of a hydroxide of an alkali metal or alkaline earth metal (e.g., sodium hydroxide, potassium hydroxide, or magnesium hydroxide), hydrochloric acid, or sulfuric acid, at a temperature of about 0 to 100°C, preferably about 10 to 30°C, for about 0.5 to 48 hours, preferably about 1 to 10 hours.

The amount of fluorine-containing alcohol in the obtained fluorine-containing calcium composite particles is about 1 to 50 mol%, preferably about 5 to 30 mol%. The composite particle size is about 30 to 200 nm.

The ratio of these components is such that about 10 to 1,000 parts by weight, preferably about 250 to 500 parts by weight, of fluorine-containing alcohol, and about 10 to 500 parts by weight, preferably about 10 to 50 parts by weight, of alkoxysilane are used based on 100 parts by weight of the calcium compound. When the ratio of the fluorine-containing alcohol used is less than this range, water- and oil-repellency decreases. In contrast, when the ratio of the fluorine-containing alcohol is greater than this range, dispersibility in solvents becomes poor. In addition, depending on the type of fluorine-containing alcohol, for example, when about 500 parts by weight of fluorine-containing alcohol is used, neither water- nor oil-repellency may be exhibited. Moreover, when the ratio of alkoxysilane used is less than this range, dispersibility in solvents becomes poor. In contrast, when the ratio of alkoxysilane used is greater than this range, water- and oil-repellency decreases.

The resulting reaction products, i.e., fluorine-containing calcium composite particles, are considered to have a structure in which the condensed alkoxysilane includes the calcium compound, and the fluorine-containing alcohol is further bonded via a siloxane bond, which serves as a spacer. Therefore, the fluorine-containing calcium composite particles effectively exhibit excellent water- and oil-repellency, antifouling properties, and other properties inherent in fluorine. In fact, a glass surface treated with the fluorine-containing calcium composite particles exhibits excellent water- and oil-repellency, and also has the effect of, for example, reducing the weight loss at 800°C. Moreover, the particle size of the fluorine-containing calcium composite particles, and the variation of the particle size show small values. The fluorine-containing calcium composite particles are formed as a reaction product of a fluorine-containing alcohol, alkoxysilane, and a calcium compound; however, other components are allowed to be mixed as long as the object of the present invention is not impaired.

The fluorine-containing calcium composite particles are dispersed in aqueous media mainly using water, or in various organic solvents, such as tetrahydrofuran, methanol, isopropanol, 1,2-dichloroethane, dimethylsulfoxide, or dimethylfomiamide, to a solid matters content of about 0.01 to 30 wt.%, preferably about 0.05 to 5 wt.%, thereby forming surface-treating agents, such as water- and oil- repellents and oil barriers.

The surface-treating agent dispersion may further contain other additives that are necessary for surface modification. Examples of such additives include crosslinking agents, such as melamine resin, urea resin, and blocked isocyanate; polymer extenders, silicone resin or oil, or other water repellents, such as wax; insecticides, antistatic agents, dye stabilizers, crease-preventing agents, stain blockers, and the like.

Such surface-treating agents can be effectively applied to metal, paper, film, fiber, cloth, fabric, carpet, or textile products made of filament, fiber, yarn, etc., as water- and oil- repellents; or to sliding parts of precision instruments (e.g., watches, motors, and lenses of single-lens reflex cameras) or parts adjacent to the sliding parts as surface-treating agents, such as oil barriers, for preventing leakage of lubricating oil from sliding surfaces to neighboring parts. As the application method, coating, dipping, spraying, padding, roll coating, or a combination of these methods is generally used. For example, the surface-treating agent is used as a pad bath having a solid matters content of about 0.1 to 10 wt.%. A material to be treated is padded in the pad bath, and the excessive liquid is removed by squeeze rolls, followed by drying, so that the amount of the polymer attached to the material is about 0.01 to 10 wt.%. Subsequently, drying is generally carried out at a temperature of about 100 to 200°C for about 1 minute to about 2 hours, although it depends on the type of material to be treated. Thus, the water- and oil-repellent treatment is completed.

EXAMPLES

The following describes the present invention with reference to Examples.

Example 1

To a solution in which 500 mg of fluorine-containing alcohol of the formula: $CF_3(CF_2)_3CH_2(CF_2)_5(CH_2)_2OH$ [DTFA-103; $C_4F_9(CH_2CF_2)(CF_2CF_2)_2(CH_2CH_2)OH$] was dissolved in 5 ml of methanol, a dispersion of 222 mg of calcium chloride $CaCl_2$ (produced by Wako Pure Chemical Industries, Ltd.) in 4 ml of methanol was added, and the mixture was stirred with a magnetic stirrer for 10 minutes.

To the resulting mixture, 5 ml of 25 wt.% aqueous ammonia was added and stirred at room temperature overnight. Then, the solvent was removed under reduced pressure, and the residue was redispersed in 5 ml of methanol and allowed to stand overnight. Thereafter, the solid product was separated by centrifugation and washed several times with methanol. The obtained powder was dried under reduced pressure at 50°C for 24 hours, thereby obtaining 130.0 mg of white powdery fluorine-containing calcium composite particles (yield: 18%).

The yield is expressed as the amount of produced fluorine-containing calcium composite particles relative to the total amount of starting materials to be used (fluorine-containing alcohol + calcium compound).

The particle size of the obtained white powdery fluorine-containing calcium composite particles, and the variation of the particle size were measured in a methanol dispersion having a solid matters content of 1 g/L at 25°C by a dynamic light scattering (DLS) method (using DLS-6000HL, produced by Otsuka Electronics Co., Ltd.). Further, thermogravimetric analysis (TGA) was performed before calcining and after calcining up to 800°C. The heating rate in this case was 10°C/min. In the fluorine-containing calcium composite particles after calcining, the crystal structure of calcium fluoride was confirmed by comparison with diffraction patterns for identification by X-ray diffraction measurement using 2 θ/deg as the horizontal axis.

Examples 2 and 3, and Comparative Example 1

In Example 1, the amount of 25 wt.% aqueous ammonia was changed in various amounts.

Examples 4 to 6 and Comparative Example 2

In Examples 1 to 3 and Comparative Example 1, 328 mg of calcium nitrate $Ca(NO_3)_2$ (produced by Wako Pure Chemical Industries, Ltd.) was used in place of calcium chloride.

Examples 7 to 9 and Comparative Example 3

In Examples 1 to 3 and Comparative Example 1, 461 mg of calcium methanesulfonate $Ca(OSO_3CH_3)_2$ (produced by Tokyo Chemical Industry Co., Ltd.) was used in place of calcium chloride.

Examples 10 to 13

In Examples 1 to 3 and Comparative Example 1, 148 mg of calcium hydroxide $Ca(OH)_2$ (produced by Wako Pure Chemical Industries, Ltd.) was used in place of calcium chloride.

Table 1 below shows the results obtained in the above Examples and Comparative Examples.

Table 1

| Example | 25% aqueous $NH_3$ (ml) | Ca compound (mg) | | Recovery amount(mg) | Yield % | Fluorine-containing calcium composite particle size (nm) | |
|---|---|---|---|---|---|---|---|
| | | | | | | Before calcining | After calcining up to 800°C |
| Ex. 1 | 5 | $CaCl_2$ | 222 | 130.0 | 18 | 37.8±8.5 | 70.5±14.2 |
| Ex. 2 | 3 | $CaCl_2$ | 222 | 122.7 | 17 | 22.5±2.7 | 51.2±6.6 |
| Ex. 3 | 1 | $CaCl_2$ | 222 | 72.2 | 10 | 28.9±3.4 | 38.3±7.2 |
| Comp. Ex. 1 | - | $CaCl_2$ | 222 | 0 | 0 | - | |
| Ex. 4 | 5 | $Ca(NO_3)_2$ | 328 | 157.4 | 19 | 109.0±20.0 | 34.1±6.0 |
| Ex. 5 | 3 | $Ca(NO_3)_2$ | 328 | 132.5 | 16 | 47.3±8.5 | 58.6±7.2 |
| Ex. 6 | 1 | $Ca(NO_3)_2$ | 328 | 66.3 | 8 | 97.7±18.9 | 26.6±3.8 |
| Comp. Ex. 2 | - | $Ca(NO_3)_2$ | 328 | 0 | 0 | - | |
| Ex. 7 | 5 | $Ca(OSO_3Me)_2$ | 461 | 57.6 | 6 | 33.7±7.1 | 17.2±3.2 |
| Ex. 8 | 3 | $Ca(OSO_3Me)_2$ | 461 | 57.6 | 6 | 37.8±6.3 | 17.9±4.1 |
| Ex. 9 | 1 | $Ca(OSO_3Me)_2$ | 461 | 9.6 | 1 | - | |
| Comp. Ex. 3 | - | $Ca(OSO_3Me)_2$ | 461 | - | 0 | - | |
| Ex. 10 | 5 | $Ca(OH)_2$ | 148 | 103.7 | 16 | 31.2±8.0 | 35.9±8.4 |
| Ex. 11 | 3 | $Ca(OH)_2$ | 148 | 90.7 | 14 | 29.5±7.9 | 35.4±8.6 |
| Ex. 12 | 1 | $Ca(OH)_2$ | 148 | 77.8 | 12 | 31.4±7.5 | 27.2±6.1 |
| Ex. 13 | - | $Ca(OH)_2$ | 148 | 129.6 | 20 | 34.1±8.2 | 29.3±5.9 |

Comparative Examples 4 to 11

When 116 mg of each following fluorine-containing alcohol was used as the fluorine-containing alcohol in Example 3, in which 1 ml of 25 wt.% aqueous ammonia and 222 mg of calcium chloride were used, no fluorine-containing calcium composite particle was obtained in any of the cases.

Comparative Example 4:  $CF_3CF_2CF_2OCF(CF_3)CF_2OCF(CF_3)CH_2OH[PO-3-OH]$

Comparative Example 5:  $CF_3(CF_2)_2O[CF(CF_3)CF_2O]_4CF(CF_3)CH_2OH[PO-6-OH]$

Comparative Example 6: $HOCH_2[CF(CF_3)OCF_2]_2CF_2OCF(CF_3)CH_2OH$ [OXF3PO-OH]

Comparative Example 7: $HOCH_2CF(CF_3)[OCF_2CF(CF_3)]_nO(CF_2)_2O-[CF(CF_3)CF_2O]_mCF(CF_3)CH_2OH[n + m = 6;$ OXF8PO-OH]

Comparative Example 8: $HOCH_2CF(CF_3)[OCF_2CF(CF_3)]_nO(CF_2)_2O-[CF(CF_3)CF_2O]_mCF(CF_3)CH_2OH[n + m = 12;$ OXF14PO-OH]

Comparative Example 9: $CF_3(CF_2)_3(CH_2)_2OH[FA-4; C_2F_5(CF_2CF_2)(CH_2CH_2)OH]$

Comparative Example 10: $CF_3(CF_2)_5(CH_2)_2OH$ [FA-6; $C_2F_5(CF_2CF_2)_2(CH_2CH_2)OH]$

Comparative Example 11: $CF_3(CF_2)_7(CH_2)_2OH$ [FA-8; $C_2F_5(CF_2CF_2)_3(CH_2CH_2)OH]$

Examples 14 to 17

Prepared glass slides were dipped in methanol dispersions (particle concentration: 5 g/L) of the fluorine-containing calcium composite particles before calcining obtained in Examples 2, 5, 8, and 11, and then dried at room temperature. Droplets (4 $\mu$l) were gently brought into contact with the obtained thin layer surfaces at room temperature, and the contact angle (unit: °) of the droplets adhering to n-dodecane or water was measured by the $\theta/2$ method using a contact angle meter (DropMaster 300, produced by Kyowa Interface Science Co., Ltd.). The contact angle with water was measured with time. Table 2 below shows the obtained results.

Table 2

| Ex. | Composite | Dodecane | Water (elapsed time: min) | | | | | | |
|-----|-----------|----------|---|----|----|----|----|----|----|
| | | | 0 | 5 | 10 | 15 | 20 | 25 | 30 |
| 14 | Ex. 2 | 60 | 38 | 6 | 0 | - | - | - | - |
| 15 | Ex. 5 | 63 | 46 | 0 | - | - | - | - | - |
| 16 | Ex. 8 | 91 | 73 | 9 | 0 | - | - | - | - |
| 17 | Ex. 11 | 80 | 111 | 22 | 17 | 13 | 7 | 0 | - |

Example 18

To a solution in which 500 mg of fluorine-containing alcohol of the formula: $CF_3(CF_2)_3CH_2(CF_2)_5(CH_2)_2OH$ [DTFA-103; $C_4F_9(CH_2CF_2)(CF_2CF_2)_2(CH_2CH_2)OH]$ was dissolved in 5 ml of methanol, 100 mg of particulate calcium carbonate (produced by Shiraishi Calcium Kaisha, Ltd.; average particle size: 80 nm) was added. While the mixture was stirred with a magnetic stirrer, 0.13 ml of tetraethoxysilane (produced by Tokyo Chemical Industry Co., Ltd.; density: 0.93 g/ml) was added and stirred for 10 minutes.

To the resulting mixture, 2 ml of 25 wt.% aqueous ammonia was added and reacted at room temperature for 3 hours. Then, the solvent was removed under reduced pressure, and the residue was redispersed in 5 ml of methanol and allowed to stand overnight. Thereafter, the solid product was separated by centrifugation and washed several times with methanol. The obtained powder was dried under reduced pressure at 50°C for 24 hours, thereby obtaining 137 mg of white powdery fluorine-containing calcium composite particles (yield: 19%).

The yield was calculated by the following formula on the assumption that tetraalkoxysilane underwent a self-condensation reaction to form three-dimensional siloxane bonds Si-O and generate a -O-Si-O- [$SiO_2$] skeleton.

$$Yield\ (\%) = A/[B + C + (D \times E)] \times 100$$

A: weight of produced composite (g)
B: weight of fluorine-containing alcohol (g)
C: weight of calcium compound (g)
D: volume of tetraalkoxysilane (ml)
E: density of tetraalkoxysilane (g/ml)

Examples 19 to 25

In Example 18, the amount of fluorine-containing alcohol DTFA-103 was changed in various amounts.

Examples 26 to 33

In Examples 18 to 25, the same amount of $CF_3(CF_2)_3(CH_2)_2OH$ [FA-4; $C_2F_5(CF_2CF_2)(CH_2CH_2)OH$] was used as the fluorine-containing alcohol.

Examples 34 to 41

In Examples 18 to 25, the same amount of $CF_3(CF_2)_5(CH_2)_2OH$ [FA-6 ; $C_2F_5(CF_2CF_2)_2(CH_2CH_2)OH$] was used as the fluorine-containing alcohol.

Examples 42 to 49

In Examples 18 to 25, the same amount of $CF_3(CF_2)_7(CH_2)_2OH$ [FA-8 ; $C_2F_5(CF_2CF_2)_3(CH_2CH_2)OH$] was used as the fluorine-containing alcohol.

Examples 50 to 57

In Examples 18 to 25, the same amount of $CF_3CF_2CF_2OCF(CF_3)CF_2OCF(CF_3)CH_2OH$[PO-3-OH] was used as the fluorine-containing alcohol.

Examples 58 to 65

In Examples 18 to 25, the same amount of $CF_3(CF_2)_2O[CF(CF_3)CF_2O]_4CF(CF_3)CH_2OH$[PO-6-OH] was used as the fluorine-containing alcohol.

Examples 66 to 71

In Examples 18 and 21 to 25, the same amount of $HOCH_2[CF(CF_3)OCF_2]_2CF_2OCF(CF_3)CH_2OH$ [OXF3PO-OH] was used as the fluorine-containing alcohol.

Examples 72 to 77

In Examples 18 and 21 to 25, the same amount of $HOCH_2CF(CF_3)[OCF_2CF(CF_3)]_nO(CF_2)_2O[CF(CF_3)CF_2O]_mCF(CF_3)CH_2OH$ [n+m=6 ; OXF8PO-OH] was used as the fluorine-containing alcohol.

Examples 78 to 82

In Examples 18 and 22 to 25, the same amount of $HOCH_2CF(CF_3)[OCF_2CF(CF_3)]_nO(CF_2)_2O[CF(CF_3)CF_2O]_mCF(CF_3)CH_2OH$ [n+m=12 ; OXF14PO-OH] was used as the fluorine-containing alcohol.

Table 3 below shows the measurement results obtained in the above Examples. In some of the Examples in which 500 mg or 300 mg of fluorine-containing alcohol was used, the amount of calcium carbonate was changed to 200 mg or 30 mg. In the table, the amount of fluorine-containing alcohol expressed by [500] indicates that the amount of calcium carbonate used in that case was changed to 200 mg. Further, the amount of fluorine-containing alcohol expressed by (300) indicates that the amount of calcium carbonate used in that case was changed to 30 mg.

Table 3

Fluorine-containing calcium composite particle size (nm)

| Ex. | Alcohol (mg) | Composite (mg) | Yield (%) | Before calcining | After calcining up to 800°C | Weight loss (%) |
|---|---|---|---|---|---|---|
| [DTF A-103] | | | | | | |
| 18 | 500 | 137 | 19 | 79.1±13.5 | 61.4±4.0 | 15 |
| 19 | [500] | 246 | 30 | 80.3±17.6 | 90.5±15.2 | 32 |
| 20 | 400 | 155 | 25 | 55.8±15.8 | 63.9±5.9 | 29 |
| 21 | (300) | 81 | 18 | 84.6±17.6 | 90.5±15.2 | 26 |
| 22 | 200 | 169 | 40 | 93.9±16.4 | 141.4±27.1 | 24 |
| 23 | 150 | 182 | 49 | 82.1±14.8 | 80.6±15.8 | 27 |
| 24 | 100 | 180 | 56 | 58.8±20.0 | 46.6±9.6 | 30 |
| 25 | 70 | 205 | 70 | 80.6±15.6 | 73.8±14.9 | 30 |
| [FA-4] | | | | | | |
| 26 | 500 | 166 | 23 | 69.8±15.6 | 41.8±10.4 | 36 |
| 27 | [500] | 263 | 32 | 83.0±15.1 | 55.6±5.7 | 38 |
| 28 | 400 | 161 | 26 | 148.8±28.4 | 123.6±23.0 | 28 |
| 29 | (300) | 90 | 20 | 77.6±15.8 | 46.6±10.8 | 28 |
| 30 | 200 | 186 | 44 | 148.8±28.4 | 123.6±23.0 | 36 |
| 31 | 150 | 190 | 51 | 84.3±13.4 | 85.5±17.0 | 38 |
| 32 | 100 | 200 | 62 | 155.0±24.3 | 145.1±30.0 | 35 |
| 33 | 70 | 205 | 70 | 70.2±11.4 | 52.7±10.6 | 31 |
| [FA-6] | | | | | | |
| 34 | 500 | 152 | 21 | 82.4±18.9 | 57.3±13.1 | 38 |
| 35 | [500] | 254 | 31 | 68.8±13.6 | 62.9±10.4 | 39 |
| 36 | 400 | 155 | 25 | 50.7±11.3 | 39.0±8.2 | 36 |
| 37 | (300) | 90 | 20 | 54.6±12.1 | 47.3±10.3 | 27 |
| 38 | 200 | 177 | 42 | 103.9±14.9 | 73.8±13.0 | 34 |
| 39 | 150 | 186 | 50 | 182.8±34.4 | 126.1±24.2 | 36 |
| 40 | 100 | 180 | 56 | 137.6±14.5 | 137.6±24.6 | 34 |
| 41 | 70 | 202 | 69 | 69.8±14.3 | 51.0±10.6 | 32 |
| [FA-8] | | | | | | |
| 42 | 500 | 159 | 22 | 51.3±11.6 | 35.6±8.3 | 35 |
| 43 | [500] | 271 | 33 | 68.6±13.0 | 57.7±7.7 | 39 |
| 44 | 400 | 168 | 27 | 93.5±17.3 | 99.0±18.8 | 34 |
| 45 | (300) | 86 | 19 | 80.7±16.4 | 54.4±12.3 | 28 |
| 46 | 200 | 173 | 41 | 88.4±13.9 | 54.7±11.8 | 33 |
| 47 | 150 | 190 | 51 | 88.1±11.8 | 64.4±10.8 | 35 |
| 48 | 100 | 203 | 63 | 53.6±7.8 | 47.5±12.3 | 31 |
| 49 | 70 | 207 | 71 | 85.1±15.4 | 56.6±10.4 | 35 |
| [PO-3-OH] | | | | | | |
| 50 | 500 | 166 | 23 | 70.1±14.1 | 47.1±10.3 | 33 |
| 51 | [500] | 271 | 33 | 24.9±5.8 | 25.8±5.6 | 39 |
| 52 | 400 | 161 | 26 | 54.6±12.1 | 75.0±13.1 | 36 |
| 53 | (300) | 86 | 19 | 70.9±15.7 | 60.8±13.5 | 25 |
| 54 | 200 | 177 | 42 | 77.2±11.1 | 45.9±10.5 | 34 |
| 55 | 150 | 175 | 47 | 54.0±17.6 | 20.7±2.9 | 32 |
| 56 | 100 | 190 | 59 | 59.3±11.5 | 47.5±12.3 | 37 |
| 57 | 70 | 240 | 82 | 84.6±15.5 | 42.5±6.8 | 34 |

(continued)

[PO-6-OH]

| 58 | 500 | 144 | 20 | 80.3±19.1 | 31.4±7.4 | 41 |
|---|---|---|---|---|---|---|
| 59 | [500] | 263 | 32 | 36.6±9.1 | 48.6±4.9 | 38 |
| 60 | 400 | 168 | 27 | 55.0±14.3 | 42.7±10.5 | 26 |
| 61 | (300) | 95 | 21 | 55.6±13.8 | 48.7±11.3 | 25 |
| 62 | 200 | 182 | 43 | 53.6±12.6 | 33.3±3.8 | 33 |
| 63 | 150 | 190 | 51 | 57.3±12.4 | 24.6±2.4 | 33 |
| 64 | 100 | 200 | 62 | 80.3±11.5 | 63.0±5.9 | 35 |
| 65 | 70 | 237 | 81 | 72.5±7.1 | 58.1±14.8 | 33 |

[OXF3PO-OH]

| 66 | 500 | 166 | 23 | 53.1±12.6 | 38.7±10.2 | 36 |
|---|---|---|---|---|---|---|
| 67 | (300) | 95 | 21 | 52.8±11.6 | 38.4±10.6 | 27 |
| 68 | 200 | 190 | 45 | 108.5±16.4 | 82.4±13.8 | 34 |
| 69 | 150 | 197 | 53 | 53.8±3.5 | 34.1±3.4 | 36 |
| 70 | 100 | 209 | 65 | 108.4±17.7 | 82.2±7.6 | 32 |
| 71 | 70 | 207 | 71 | 84.2±12.2 | 72.7±7.2 | 32 |

[OXF8PO-OH]

| 72 | 500 | 166 | 23 | 40.2±13.5 | 108.4±20.0 | 39 |
|---|---|---|---|---|---|---|
| 73 | (300) | 95 | 21 | 52.7±11.4 | 71.2±15.7 | 25 |
| 74 | 200 | 194 | 46 | 82.1±14.5 | 64.8±16.6 | 37 |
| 75 | 150 | 201 | 54 | 50.8±10.8 | 44.9±5.2 | 35 |
| 76 | 100 | 184 | 57 | 73.4±17.0 | 37.6±5.5 | 34 |
| 77 | 70 | 210 | 72 | 114.4±21.2 | 115.3±21.4 | 34 |

[OXF14PO-OH]

| 78 | 500 | 281 | 39 | 104.7±18.7 | 89.0±13.1 | 71 |
|---|---|---|---|---|---|---|
| 79 | 200 | 274 | 65 | 75.3±15.8 | 71.0±14.5 | 59 |
| 80 | 150 | 208 | 56 | 73.4±13.1 | 55.1±12.7 | 40 |
| 81 | 100 | 213 | 66 | 84.4±15.2 | 54.7±12.4 | 34 |
| 82 | 70 | 234 | 80 | 51.2±4.4 | 42.5±4.3 | 38 |

Examples 83 to 100 and Comparative Examples 12 to 16

Using methanol dispersions (particle concentration: 5 g/L) of the fluorine-containing calcium composite particles before calcining obtained in some of the above Examples, the contact angle (unit: °) of droplets adhering to n-dodecane or water was measured by the above-mentioned measurement method. Table 4 below shows the obtained results. In Comparative Example 15, only particulate calcium carbonate was used. The measured values of Comparative Example 16 were obtained from calcium composite particles (weight loss: 29%) produced without using a fluorine-containing alcohol in Example 18.

Table 4

| Example | Composite | Contact angle | | Evaluation |
|---|---|---|---|---|
| | | Dodecane | Water | |
| Ex. 83 | Ex. 18 | 128 | 118 | ◎ |
| Ex. 84 | Ex. 19 | 47 | 121 | ○ |
| Ex. 85 | Ex.20 | 59 | 102 | ○ |
| Ex. 86 | Ex.25 | 38 | 31 | ○ |
| Ex. 87 | Ex. 34 | 29 | 26 | Δ |
| Ex. 88 | Ex.35 | 114 | 79 | ◎ |
| Ex. 89 | Ex. 36 | 22 | 83 | Δ |
| Ex. 90 | Ex.42 | 123 | 136 | ◎ |
| Ex. 91 | Ex.43 | 128 | 127 | ◎ |

(continued)

| Example | Composite | Contact angle | | Evaluation |
|---|---|---|---|---|
| | | Dodecane | Water | |
| Ex. 92 | Ex.44 | 130 | 124 | ◎ |
| Ex. 93 | Ex.50 | 33 | 21 | ○ |
| Ex. 94 | Ex.51 | 117 | 51 | ○ |
| Ex. 95 | Ex.52 | 97 | 51 | ○ |
| Ex. 96 | Ex. 58 | 52 | 21 | Δ |
| Ex. 97 | Ex. 59 | 61 | 54 | ○ |
| Ex. 98 | Ex. 60 | 55 | 48 | ○ |
| Ex. 99 | Ex. 72 | 53 | 83 | ○ |
| Ex. 100 | Ex. 78 | 52 | 97 | ○ |
| Comp. Ex. 12 | Ex. 26 | 28 | 13 | × |
| Comp. Ex. 13 | Ex. 28 | 14 | 0 | × |
| Comp. Ex. 14 | Ex. 66 | 28 | 15 | × |
| Comp. Ex. 15 | $CaCO_3$ | 30 | 18 | × |
| Comp. Ex. 16 | $CaCO_3\text{-}Si(OC_2H_5)_4$ | 30 | 18 | × |

Notes) Evaluation

◎: Contact angle with dodecane is 80° or more, and contact angle with water is 100° or more

○: Contact angle with dodecane is 80° or more, or contact angle with water is 100° or more Δ: Contact angle with dodecane is less than 80°, and contact angle with water is 100° or less

× : Both contact angle with dodecane and contact angle with water are lower than those of untreated product

The above results indicate that the surface tension of prepared glass slides is reduced by treating the surface of the prepared glass slides with methanol dispersions of fluorine-containing calcium composite particles.

**Claims**

1. Fluorine-containing calcium composite particles comprising an aggregate of a fluorine-containing alcohol represented by the general formula:

$$C_nF_{2n+1}(CH_2CF_2)_a(CF_2CF_2)_b(CH_2CH_2)_cOH \qquad [I]$$

wherein n is an integer of 1 to 6, a is an integer of 1 to 4, b is an integer of 0 to 3, and c is an integer of 1 to 3; and a calcium compound.

2. The fluorine-containing calcium composite particles according to claim 1, wherein the calcium compound is an inorganic calcium salt or an organic acid calcium salt.

3. A method for producing fluorine-containing calcium composite particles, comprising aggregating the fluorine-containing alcohol [I] according to claim 1 and a calcium compound using an alkaline or acid catalyst.

4. The method for producing fluorine-containing calcium composite particles according to claim 3, wherein aqueous ammonia is used as the alkaline catalyst.

5. The method for producing fluorine-containing calcium composite particles according to claim 3, wherein calcium hydroxide is used as the calcium compound and the alkaline catalyst.

6. Fluorine-containing calcium composite particles comprising a condensate of a fluorine-containing alcohol represented by the general formula:

$$R_F\text{-}A\text{-}OH \qquad [Ia]$$

or the general formula:

$$HO-A-R_F'-A-OH \qquad [Ib]$$

wherein $R_F$ is a liner or branched perfluoroalkyl group that may contain an O, S, or N atom, or a polyfluoroalkyl group in which some of the fluorine atoms of the perfluoroalkyl group are replaced by hydrogen atoms; $R_F'$ is a linear or branched perfluoroalkylene group containing an O, S, or N atom; and A is an alkylene group having 1 to 6 carbon atoms; alkoxysilane, and a calcium compound.

7. The fluorine-containing calcium composite particles according to claim 6, wherein the fluorine-containing alcohol represented by the general formula [Ia] is a polyfluoroalkyl alcohol represented by the general formula:

$$C_nF_{2n+1}(CH_2CF_2)_a(CF_2CF_2)_b(CH_2CH_2)_cOH \qquad [IIa]$$

wherein n is an integer of 1 to 6, a is an integer of 1 to 4, b is an integer of 0 to 3, and c is an integer of 1 to 3.

8. The fluorine-containing calcium composite particles according to claim 6, wherein the fluorine-containing alcohol represented by the general formula [Ia] is a hexafluoropropene oxide oligomer alcohol represented by the general formula:

$$C_mF_{2m+1}O[CF(CF_3)CF_2O]_dCF(CF_3)(CH_2)_eOH \qquad [IIIa]$$

wherein m is an integer of 1 to 3, d is an integer of 0 to 100, and e is an integer of 1 to 3.

9. The fluorine-containing calcium composite particles according to claim 6, wherein the fluorine-containing alcohol represented by the general formula [Ia] is a polyfluoroalkyl alcohol represented by the general formula:

$$C_pF_{2p+1}(CH_2)_qOH \qquad [IVa]$$

or

$$C_pF_{2p}H(CH_2)_qOH \qquad [IVa']$$

wherein p is an integer of 1 to 10, and q is an integer of 1 to 6.

10. The fluorine-containing calcium composite particles according to claim 6, wherein the fluorine-containing alcohol represented by the general formula [Ib] is a perfluoroalkylene ether diol represented by the general formula:

$$HO(CH_2)_fCF(CF_3)[OCF_2CF(CF_3)]_gO(CF_2)_hO[CF(CF_3)CF_2O]_iCF(CF_3)(CH_2)_fOH \qquad [IIb]$$

wherein f is an integer of 1 to 3, g + i is an integer of 0 to 50, and h is an integer of 1 to 6.

11. The fluorine-containing calcium composite particles according to claim 6, wherein the alkoxysilane is a silane derivative represented by the general formula:

$$(R_1O)_pSi(OR_2)_q(R_3)_r \qquad [III]$$

wherein $R_1$ and $R_3$ are each a hydrogen atom, an alkyl group having 1 to 6 carbon atoms, or an aryl group; $R_2$ is an alkyl group having 1 to 6 carbon atoms or an aryl group, with the proviso that not all of $R_1$, $R_2$, and $R_3$ are aryl groups; and p + q + r is 4, with the proviso that q is not 0.

12. The fluorine-containing calcium composite particles according to claim 6, wherein the calcium compound is an inorganic calcium salt or an organic acid calcium salt.

13. A method for producing fluorine-containing calcium composite particles, comprising subjecting the fluorine-containing alcohol [Ia] or [Ib] according to claim 6 and alkoxysilane to a condensation reaction in the presence of a calcium compound using an alkaline or acid catalyst.

**14.** The method for producing fluorine-containing calcium composite particles according to claim 13, wherein the alkoxysilane is a silane derivative represented by the general formula:

$$(R_1O)_pSi(OR_2)_q(R_3)_r \qquad \text{[III]}$$

wherein $R_1$ and $R_3$ are each a hydrogen atom, an alkyl group having 1 to 6 carbon atoms, or an aryl group; $R_2$ is an alkyl group having 1 to 6 carbon atoms or an aryl group, with the proviso that not all of $R_1$, $R_2$, and $R_3$ are aryl groups; and $p + q + r$ is 4, with the proviso that q is not 0.

**15.** A surface-treating agent comprising the fluorine-containing calcium composite particles according to claim 6 as an active ingredient.

**16.** The surface-treating agent comprising fluorine-containing calcium composite particles as an active ingredient according to claim 15, wherein the fluorine-containing alcohol represented by the general formula [Ia] is a polyfluoroalkyl alcohol represented by the general formula:

$$C_nF_{2n+1}(CH_2CF_2)_a(CF_2CF_2)_b(CH_2CH_2)_cOH \qquad \text{[IIa]}$$

wherein n is an integer of 1 to 6, a is an integer of 1 to 4, b is an integer of 0 to 3, and c is an integer of 1 to 3.

**17.** The surface-treating agent comprising fluorine-containing calcium composite particles as an active ingredient according to claim 15, wherein the fluorine-containing alcohol represented by the general formula [Ia] is a hexafluoropropene oxide oligomer alcohol represented by the general formula:

$$C_mF_{2m+1}O[CF(CF_3)CF_2O]_dCF(CF_3)(CH_2)_eOH \qquad \text{[IIIa]}$$

wherein m is an integer of 1 to 3, d is an integer of 0 to 100, and e is an integer of 1 to 3.

**18.** The surface-treating agent comprising fluorine-containing calcium composite particles as an active ingredient according to claim 15, wherein the fluorine-containing alcohol represented by the general formula [Ia] is a polyfluoroalkyl alcohol represented by the general formula:

$$C_pF_{2p+1}(CH_2)_qOH \qquad \text{[IVa]}$$

or

$$C_pF_{2p}H(CH_2)_qOH \qquad \text{[IVa']}$$

wherein p is an integer of 5 to 10, and q is an integer of 1 to 6.

**19.** The surface-treating agent comprising fluorine-containing calcium composite particles as an active ingredient according to claim 15, wherein the fluorine-containing alcohol represented by the general formula [Ib] is a perfluoroalkylene ether diol represented by the general formula:

$$HO(CH_2)_fCF(CF_3)[OCF_2CF(CF_3)]_gO(CF_2)_hO[CF(CF_3)CF_2O]_iCF(CF_3)(CH_2)_fOH \qquad \text{[IIb]}$$

wherein f is an integer 1 to 3, $g + i$ is an integer 2 to 50, and h is an integer of 1 to 6.

**20.** The surface-treating agent comprising fluorine-containing calcium composite particles as an active ingredient according to claim 16, which is used as a water- and oil-repellent.

## INTERNATIONAL SEARCH REPORT

| International application No. |
| --- |
| PCT/JP2014/055821 |

**A. CLASSIFICATION OF SUBJECT MATTER**
*C09K3/18*(2006.01)i, *C08G77/385*(2006.01)i, *C08K3/18*(2006.01)i, *C08K5/098*(2006.01)i, *C08K5/5415*(2006.01)i, *C08L71/00*(2006.01)i

According to International Patent Classification (IPC) or to both national classification and IPC

**B. FIELDS SEARCHED**

Minimum documentation searched (classification system followed by classification symbols)
C09K3/18, C08G77/385, C08K3/18, C08K5/098, C08K5/5415, C08L71/00

Documentation searched other than minimum documentation to the extent that such documents are included in the fields searched
Jitsuyo Shinan Koho          1922–1996     Jitsuyo Shinan Toroku Koho    1996–2014
Kokai Jitsuyo Shinan Koho    1971–2014     Toroku Jitsuyo Shinan Koho    1994–2014

Electronic data base consulted during the international search (name of data base and, where practicable, search terms used)
CAplus/REGISTRY(STN), JSTPlus/JMEDPlus/JST7580(JDreamIII)

**C. DOCUMENTS CONSIDERED TO BE RELEVANT**

| Category* | Citation of document, with indication, where appropriate, of the relevant passages | Relevant to claim No. |
| --- | --- | --- |
| A | JP 2007-277516 A  (Ishihara Chemical Co., Ltd.), 25 October 2007 (25.10.2007), claims (Family: none) | 1-20 |
| A | JP 05-285373 A  (Kao Corp.), 02 November 1993 (02.11.1993), claims (Family: none) | 1-20 |
| A | WO 2007/105633 A1  (Unimatec Co., Ltd.), 20 September 2007 (20.09.2007), claims; paragraph [0001] & JP 2010-189449 A      & JP 4674604 B & US 2009/0036706 A1    & EP 1995228 A1 & EP 2228356 A1 | 1-20 |

☒ Further documents are listed in the continuation of Box C.       ☐ See patent family annex.

| | |
| --- | --- |
| * Special categories of cited documents:<br>"A" document defining the general state of the art which is not considered   to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| 02 June, 2014 (02.06.14) | 10 June, 2014 (10.06.14) |

| Name and mailing address of the ISA/<br>Japanese Patent Office | Authorized officer |
| --- | --- |
| Facsimile No. | Telephone No. |

Form PCT/ISA/210 (second sheet) (July 2009)

## INTERNATIONAL SEARCH REPORT

International application No.

PCT/JP2014/055821

C (Continuation).  DOCUMENTS CONSIDERED TO BE RELEVANT

| Category* | Citation of document, with indication, where appropriate, of the relevant passages | Relevant to claim No. |
|---|---|---|
| A | WO 2007/080949 A1  (Unimatec Co., Ltd.),<br>19 July 2007 (19.07.2007),<br>claims; paragraph [0002]<br>& JP 2007-186454 A       & US 2009/0171127 A1<br>& EP 1972610 A1 | 1-20 |
| A | US 3574770 A  (T.O.Paine),<br>13 April 1971 (13.04.1971),<br>claims<br>(Family: none) | 1-20 |
| A | JP 2009-073799 A  (Ishihara Chemical Co., Ltd.),<br>09 April 2009 (09.04.2009),<br>claims<br>(Family: none) | 1-20 |
| A | JP 2012-188635 A  (Ishihara Chemical Co., Ltd.,<br>Hirosaki University),<br>04 October 2012 (04.10.2012),<br>claims<br>(Family: none) | 1-20 |
| A | JP 2010-138156 A  (Hirosaki University,<br>Ishihara Chemical Co., Ltd.),<br>24 June 2010 (24.06.2010),<br>claims<br>(Family: none) | 1-20 |
| A | JP 2010-209280 A  (Mitsubishi Materials Corp.,<br>Mitsubishi Materials Electronic Chemicals Co.,<br>Ltd., Hirosaki University),<br>24 September 2010 (24.09.2010),<br>claims<br>(Family: none) | 1-20 |
| A | JP 06-285363 A  (Shin-Etsu Chemical Co., Ltd.),<br>11 October 1994 (11.10.1994),<br>claims<br>(Family: none) | 1-20 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2180984 A
- JP 2012233157 A
- JP 4674604 B
- WO 2007080949 A1
- JP 2008038015 A
- US 3574770 P